# EUROPÄISCHE PATENTSCHRIFT

(11) **EP 1 371 353 B1**
(45) Veröffentlichungstag und Bekanntmachung des Hinweises auf die Patenterteilung: **06.08.2008**
(21) Anmeldenummer: 03005556.0
(22) Anmeldetag: 12.03.2003
(51) Int. Cl.: A61K 8/898, A61K 8/41, A61K 8/73, A61K 8/81, A61Q 5/10, A61Q 5/12

(54) **Verdicktes kationisches kosmetisches Mittel Inulin enthaltend**
Thickened cationic cosmetic composition comprising inulin
Composition cosmétique épaissie comprenant de l'inuline

(30) Priorität: 15.06.2002 DE 10226818
(43) Veröffentlichungstag der Anmeldung: 17.12.2003
(73) Patentinhaber: Wella Aktiengesellschaft, 64274 Darmstadt (DE)
(72) Erfinder: Pfaffernoschke, Matthisa, Dr., 64807 Dieburg (DE); Buntschu, Cindy, 1712 Tafers (CH); Allwohn, Jürgen, Dr., 65558 Burgschwalbach (DE); Steinbrecht, Karin, Dr., 64372 Ober-Ramstadt (DE); Birkel, Susanne, Dr., 64285 Darmstadt (DE)

(56) Entgegenhaltungen:
- EP-A- 1 046 390
- EP-A- 1 133 973
- EP-A- 1 174 118
- WO-A-01/19204
- WO-A-02/055036
- DE-A- 10 004 644
- DE-A- 19 617 605
- FR-A- 2 795 953
- US-A- 4 364 837
- PATENT ABSTRACTS OF JAPAN vol. 1998, no. 08, 30. Juni 1998 (1998-06-30) & JP 10 072312 A (SHISEIDO CO LTD), 17. März 1998 (1998-03-17)

## Beschreibung

Gegenstand der Erfindung ist verdicktes kationisches kosmetisches Mittel, insbesondere ein Haarpflegemittel, welches insbesondere als Haarkur oder als Haarspülung anwendbar ist, vorzugsweise in Form einer Creme vorliegt und einen hohen Gehalt an Inulin als Verdicker sowie kationische haarpflegenden Stoffe aufweist.

Gängige Haarpflegemittel liegen heutzutage in Form einer Emulsion vor, wobei meistens ein Fettalkohol durch eine quaternisierte Ammoniumverbindung mit einem liphophilen Ende in Wasser emulgiert wird. Das Kationentensid dient hierbei sowohl als Emulgator und als Pflegekomponente. Durch geeignete Fettalkohol/Kationentensid-Verhältnisse lassen sich Viskosität und Pflegeeigenschaften einstellen.

Übliche haarkonditionierende Präparate wie Rinse-off Kuren oder Leave-on Treatments sind in der Regel auf der Basis von wässrigen Emulsionen formuliert. Wesentliche Inhaltsstoffe sind kationaktive Substanzen wie z. B. kationische Tenside, hydrophobe Substanzen wie z. B. Fettalkohole und andere Ölkomponenten, Emulgatoren, sowie weitere spezifische Wirk- und Duftstoffe. Die wichtigsten Bestandteile sind dabei kationische Tenside, Fettalkohole und Emulgatoren. Hauptaufgaben der Konditioniermittel sind die Verbesserung der Frisierbarkeit, der Kämmbarkeit, des Glanzes und des Griffs des behandelten Haares. Die behandelten Haare fühlen sich häufig etwas schwerer und belasteter an, was nicht immer erwünscht ist. Die herkömmlichen Haarkurmittel sind auf Basis von verdickend wirkenden Polymeren wie z.B. Cellulosederivaten (Handelsnamen Natrosol®, Methocel®), hochmolekularen Chitosanderivaten (Handelsname Kytamer® PC), komplexen Polysacchariden (Handelsnamen Karaya Gum, Tragant, Jaguar® Typen, Keltrol® Typen) und Acrylsäurepolymerisaten hergestellt. Alle diese beschriebenen Haarkurmittel haben den großen Nachteil, dass die Pflegewirkung so schwach ist, dass sie bei weitem nicht diejenige eines klassischen Haarkurmittels auf Basis von Mischungen von Fettalkoholen und quaternären Tensiden erreicht. Diese, nach dem Stand der Technik bekannten Haarkurmittel verkaufen sich deshalb auf dem Markt deutlich schlechter als die Standardkuren.

In der nicht vorveröffentlichten älteren Anmeldung WO 02/055036 A2 sind in den Beispielen 4 und 6 Shampoos mit einem Gehalt an 15 Gew.% Inulin und 4 Gew.% des kationischen Tensids Palmitylamidopropyltrimethylammoniumchlorid sowie 0,1 Gew.% eines Homopolymers von Ethyltrimethylammoniummethycrylat chlorid bzw. POLYQUATERNIUM-10 beschrieben.

Die DE 19617605 A1 beschreibt ein Verfahren zur Herstellung einer wärmebehandelten Polyfructosecreme mit erhöhter Festigkeit, die 10 bis 50 Gew.% Polyfructose enthält, wobei die Polyfructose Inulin sein kann, für die Verwendung in Lebensmitteln aber auch als Basis in kosmetischen Mitteln, wie Hautcremes und Zahncremes.

Aus der WO 01/19204 A1 sind zum Verzehr bestimmte Mittel wie Lutschtabletten und Kaugummi bekannt, welche 40 bis 90% Inulin und 0,02 bis 0,3 einer zweiwertigen Zink- oder Kupferverbindung enthalten, zur Kontrolle des Mungeruchs ohne dass dabei die Kariesbildung gefördert wird. Die Wirkung soll darauf beruhen, dass Inulin den Enzymen der Mundhöhle widersteht.

Die FR 2795953 A1 kosmetische Mittel mit einem Gehalt an einer Kombination aus einem Fructanderivat mit Aminogruppe wie z. B. Trimethyl-hydroxypropylammonium-inulin und einem konditionierenden Wirkstoff wie z. B. einem Öl, Fett, kationischen Polymer oder kationischen Tensid.

In der EP 1133973 A1 ist offenbart, dass Haarkonditionierungsmittel in der Regel kationische Wirkstoffe, insbesondere quaternäre langkettige Ammoniumverbindungen und/oder Polymere mit quaternären Stickstoffatomen, enthalten und insbesondere als Lösungen, Öl-in-Wasser-Emulsionen, Gele oder Lotionen eingesetzt werden.

Aus der DE-OS 10004644 ist es bekannt; tensidhaltige kosmetische Mittel mit geringen Mengen von 0,1 bis 10 Gew.%, vorzugsweise 1 bis 5 Gew.%, eines alkylierten oder alkoxylierten Inulinderivates zu verdicken. Alkylierte oder alkoxylierte Inulinderivate sind physiologisch schlechter verträglich, als das Inulin selbst. Wenn diese Zubereitungen kationische Stoffe enthalten, ist darin zudem auch mindestens die selbe Menge eines Fettalkohols enthalten. Fettalkohole haben jedoch den Nachteil, dass sie das Haar belasten und ihm ein fettiges Aussehen verleihen.

Aufgabe der vorliegenden Erfindung war es daher, bei gleichbleibenden Pflegeeigenschaften auf Fettalkohol zu Verzichten und ein Mittel auf der Basis eines einen physiologisch sehr gut verträglichen Verdickers zur Verfügung zu stellen.

Überraschend wurde gefunden, dass eine Lösungen von mindestens 12 Gew.% Inulin in Wasser eine Creme mit thixotropem Fliessverhalten (kolloidale Lösung) bildet, welche mit kationischen Stoffen verträglich ist. Dieser Ansatz wird in der hier beschriebenen Erfindung genutzt, um neue Grundlagen für kationische Haarpflegeprodukte herzustellen. Es ist bekannt, Inuline in der Lebensmittelindustrie als Ballaststoffe in "light-Produkten" einzusetzen, da sie den fettarmen Produkten einen "gehaltvolleren" Eindruck verleihen.

Es bestand somit die Aufgabe, ein Mittel zur Verfügung zu stellen, welches die typischen, an ein Haarkonditioniermittel zu stellenden Anforderungen hinsichtlich Haarkonditionierung erfüllt und dem Haar möglichst einen weniger schweren und belasteten Eindruck verleiht, als nach einer Behandlung mit einem herkömmlichen Haarkonditioniermittel.

Es wurde nun gefunden, dass die Aufgabe gelöst wird durch ein Mittel nach Anspruch 1.

Beispiele für bevorzugte kationische Tenside sind die Chloride oder Bromide von Alkyldimethylbenzylammoniumsalzen, Alkyltrimethylammoniumsalze, beispielsweise Cetyltrimethylammoniumchlorid oder -bromid, Tetradecyltrimethylammoniumchlorid oder -bromid, Alkyldimethylhydroxyethylammoniumchloride oder -bromide, die Dialkyldimethylammoniumchloride oder -bromide, Alkylpyridiniumsalze, beispielsweise Lauryl- oder Cetylpyridiniumchlorid, Verbindungen mit kationischem Charakter wie Aminoxide, beispielsweise Alkylmethylaminoxide oder Alkylaminoethyldimethylaminoxide. Besonders bevorzugt ist Cetyltrimethylammoniumchlorid, das beispielsweise in Form einer 26prozentigen wässrigen Lösung unter der Handelsbezeichnung Dehyquart^{®} A von der Firma Henkel KGaA, Düsseldorf/Deutschland und unter der Handelsbezeichnung Genamin^{®} CTAC von der Firma Hoechst AG, Frankfurt/Deutschland sowie in Form einer 50prozentigen Lösung in Isopropanol unter der Handelsbezeichnung Arquad^{®} 16-50 von der Firma Akzo Chemicals GmbH, Düren/Deutschland vertrieben wird.

Bei den geeigneten kationaktiven Polymeren handelt es sich vorzugsweise um haarfestigende oder um haarkonditionierende Polymere. Die kationischen Polymere können Homo- oder Copolymere sein, wobei die quaternären Stickstoffgruppen entweder in der Polymerkette oder vorzugsweise als Substituent an einem oder mehreren der Monomeren enthalten sind. Die Ammoniumgruppen enthaltenden Monomere können mit nicht kationischen Monomeren copolymerisiert sein. Geeignete kationische Monomere sind ungesättigte, radikalisch polymerisierbare Verbindungen, welche mindestens eine kationische Gruppe tragen, insbesondere ammoniumsubstituierte Vinylmonomere wie z.B.

Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alyklvinylpyrrolidon Salze. Die Alkylgruppen dieser Monomere sind vorzugsweise niedere Alkylgruppen wie z.B. C1- bis C7-Alkylgruppen, besonders bevorzugt C1- bis C3-Alkylgruppen.

Die Ammoniumgruppen enthaltenden Monomere können mit nicht kationischen Monomeren copolymerisiert sein. Geeignete Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylcaprolactam, Vinylpyrrolidon, Vinylester, z. B. Vinylacetat, Vinylalkohol, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Ein geeignetes Polymer mit quaternären Amingruppen ist beispielsweise das im CTFA Cosmetic Ingredient Dictionary unter den Bezeichnungen Polyquaternium beschriebene Polymer Methylvinylimidazoliumchlorid/Vinylpyrrolidon Copolymer (Polyquaternium-16).

Besonders bevorzugte kationaktive Stoffe sind Chitosan, Chitosansalze und Chitosan-Derivate. Bei den erfindungsgemäß einzusetzenden Chitosanen handelt es sich um vollständig oder partiell deacetylierte Chitine. Zur Herstellung von Chitosan geht man vorzugsweise von dem in den Schalenresten von Krustentieren enthaltenem Chitin aus, welches als billiger und natürlicher Rohstoff in großen Mengen zur Verfügung steht. Das Molekulargewicht des Chitosans kann über ein breites Spektrum verteilt sein, beispielsweise von 20.000 bis ca. 5 Millionen g/mol. Geeignet ist beispielsweise ein niedermolekulares Chitosan mit einem Molekulargewicht von 30.000 bis 70.000 g/mol. Vorzugsweise liegt das Molekulargewicht jedoch über 100.000 g/mol, besonders bevorzugt von 200.000 bis 700.000 g/mol. Der Deacetylierungsgrad beträgt vorzugsweise 10 bis 99%, besonders bevorzugt 60 bis 99%.

Ein geeignetes Chitosan wird beispielsweise von der Firma Kyowa Oil&Fat, Japan, unter dem Handelsnamen Flonac^{®} vertrieben. Es hat ein Molekular gewicht von 300.000 bis 700.000 g/mol und ist zu 70 bis 80% entacetyliert. Ein bevorzugtes Chitosansalz ist Chitosoniumpyrrolidoncarboxylat, welches beispielsweise unter der Bezeichnung Kytamer PC von der Firma Amerchol, USA, vertrieben wird. Das enthaltene Chitosan hat ein Molekulargewicht von ca. 200.000 bis 300.000 g/mol und ist zu 70 bis 85% entacetyliert. Als Chitosan-derivate kommen quaternierte, alkylierte oder hydroxyalkylierte Derivate, beispielsweise Hydroxyethyl- oder Hydroxybutylchitosan in Betracht.

Die Chitosane oder Chitosanderivate liegen vorzugsweise in neutralisierter oder partiell neutralisierter Form vor. Der Neutralisationsgrad für das Chitosan oder das Chitosanderivat liegt vorzugsweise bei mindestens 50%, besonders bevorzugt zwischen 70 und 100%, bezogen auf die Anzahl der freien Basengruppen. Als Neutralisationsmittel können prinzipiell alle kosmetisch verträglichen anorganischen oder organischen Säuren verwendet werden wie z. B. Ameisensäure, Weinsäure, Äpfelsäure, Milchsäure, Zitronensäure, Pyrrolidoncarbonsäure, Salzsäure u. a., von denen die Pyrrolidoncarbonsäure besonders bevorzugt ist.

Weitere geeignete kationaktive, haarpflegende Verbindungen sind kationisch modifizierte Proteinhydrolysate. Die kationisch derivatisierten Proteinhydrolysate enthalten eine oder zwei lange C8- bis C22-Alkylketten und entsprechend zwei oder eine kurze C1- bis C4-Alkylketten. Verbindungen, die eine lange Alkylkette enthalten, sind bevorzugt.

Sie sind beispielsweise bekannt unter den INCI-Bezeichnungen Lauryldimonium Hydroxypropyl Hydrolyzed Wheat Protein, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk oder Lauryldimonium Hydroxypropyl Hydrolyzed Soy Protein.

Proteine, die als Ausgangsstoffe für die Proteinhydrolysate dienen, können sowohl pflanzlicher als auch tierischer Herkunft sein. Übliche Ausgangsstoffe sind beispielsweise Keratin, Collagen, Elastin, Sojaprotein, Reisprotein, Milchprotein, Weizenprotein, Seidenprotein oder Mandelprotein. Durch die Hydrolyse entstehen Stoffmischungen mit Molmassen im Bereich von ca. 100 bis ca. 50.000. Übliche mittlere Molmassen liegen im Bereich von etwa 500 bis etwa 1000.

Geeignet und besonders bevorzugt sind kationaktive Silikonverbindungen. Diese sind mit kationischen oder kationisierbaren Gruppen substituiert. Geeignete kationaktive Silikonvberbindungen weisen entweder mindestens eine Aminogruppe oder mindestens eine Ammoniumgruppe auf. Geeignete Silikonpolymere mit Aminogruppen sind unter der INCI-Bezeichnung Amodimethicone bekannt. Hierbei handelt es sich um Polydimethylsiloxane mit Aminoalkylgruppen. Die Aminoalkylgruppen können seiten- oder endständig sein. Geeignete Aminosilikone sind solche der allgemeinen Formel

R⁵R⁶R⁷ Si-(OSiR⁸R⁹)x-(OSiR¹⁰Q)y-OSiR¹¹R¹²R¹³ (III)

R⁵, R⁶, R¹¹ und R¹² sind unabhängig voneinander gleich oder verschieden und bedeuten C1- bis C10-Alkyl, Phenyl, Hydroxy, Wasserstoff, C1- bis C10-Alkoxy oder Acetoxy, vorzugsweise C1-C4-Alkyl, besonders bevorzugt Methyl; R⁷ und R¹³ sind unabhängig voneinander gleich oder verschieden und bedeuten - (CH₂)_{d}-NH₂ mit d gleich 1 bis 6, C1- bis C10-Alkyl, Phenyl, Hydroxy, Wasserstoff, C1- bis C10-Alkoxy oder Acetoxy, vorzugsweise C1-C4-Alkyl, besonders bevorzugt Methyl;
R⁸, R⁹ und R¹⁰ sind unabhängig voneinander gleich oder verschieden und bedeuten Wasserstoff, C1- bis C20-Kohlenwasserstoff, welcher O- und N-Atome enthalten kann, vorzugsweise C1- bis C10-Alkyl oder Phenyl, besonders bevorzugt C1- bis C4-Alkyl, insbesondere Methyl;
Q bedeutet -A-NR¹⁴R¹⁵, oder -A-N⁺R¹⁴R¹⁵R¹⁶, wobei A für eine divalente C1- bis C20-Alkylenverbindungsgruppe steht, welche auch O- und N-Atome sowie OH-Gruppen enthalten kann, und R¹⁴, R¹⁵ und R¹⁶ unabhängig voneinander gleich oder verschieden sind und Wasserstoff, C1- bis C22-Kohlenwasserstoff, vorzugsweise C1- bis C-4-Alkyl oder Phenyl bedeuten. Bevorzugte Reste für Q sind -(CH₂)₃-NH₂, -(CH₂)₃NHCH₂CH₂NH₂, -(CH₂)₃OCH₂CHOHCH₂NH₂ und -(CH₂)₃N(CH₂CH₂OH)₂, -(CH₂)₃-NH₃⁺ und -(CH₂)₃OCH₂CHOHCH₂N⁺(CH₃)₂R¹⁷, wobei R¹⁷ ein C1- bis C22-Alkylrest ist, der auch OH-Gruppen aufweisen kann;
x bedeutet eine Zahl zwischen 1 und 10.000, vorzugsweise zwischen 1 und 1.000;
y bedeutet eine Zahl zwischen 1 und 500, vorzugsweise zwischen 1 und 50.

Das Molekulargewicht der Aminosilikone liegt vorzugsweise zwischen 500 und 100.000. Der Aminanteil (meq/g) liegt vorzugsweise im Bereich von 0,05 bis 2,3, besonders bevorzugt von 0,1 bis 0,5.

Geeignete Silikonpolymere mit zwei endständigen quaternären Ammoniumgruppen sind unter der INCI-Bezeichnung Quaternium-80 bekannt. Hierbei handelt es sich um Dimethylsiloxane mit zwei endständigen Aminoalkylgruppen. Geeignete quaternäre Aminosilikone sind solche der allgemeinen Formel (V)

[R¹⁸R¹⁹R²⁰N⁺-A-SiR⁵R⁶-(OSiR¹¹R¹²)ₙ-OSiR⁵R⁶-A-N⁺ R¹⁸R¹⁹R²⁰] · 2X⁻ (IV)

A hat die gleiche Bedeutung wie oben bei Formel (III) angegeben und ist, vorzugsweise -(CH₂)₃OCH₂CHOHCH₂N⁺(CH₃)₂R²¹, wobei R²¹ ein C1- bis C22-Alkylrest ist, der auch OH-Gruppen aufweisen kann;
R⁵, R⁶, R⁸ und R⁹ haben die gleiche Bedeutung wie oben bei Formel (III) angegeben und sind vorzugsweise Methyl;
R¹⁸, R¹⁹, und R²⁰ bedeuten unabhängig voneinander C1-bis C22-Alkylreste, welche Hydroxygruppen enthalten können und wobei vorzugsweise mindestens einer der Reste mindestens 10 C-Atome aufweist und die übrigen Reste 1 bis 4 C-Atome aufweisen; n ist eine Zahl von 0 bis 200, vorzugsweise von 10 bis 100. Derartige diquaternäre Polydimethylsiloxane werden von der Firma GOLDSCHMIDT/Deutschland unter den Handelsnamen Abil^{®} Quat 3270, Abil^{®} Quat 3272 und Abil^{®} Quat 3274 vertrieben.

Das kationische Polymer ist besonders bevorzugt ausgewählt aus MethylvinylimidazoliumchloridNinylpyrrolidon Copolymeren, Chitosan, Chitosansalzen und Chitosanderivaten.

Der kationische Stoff ist in dem erfindungsgemäßen Mittel vorzugsweise in einer Menge von 0,2 bis 6 Gew.%, besonders bevorzugt 0,3 bis 3 Gew.% enthalten.

Das Inulin ist in dem Mittel vorzugsweise in einer Menge von 15 bis 40 Gew.% und besonders bevorzugt in einer Menge von 18 bis 25 Gew.% enthalten.

Das Mittel enthält in einer bevorzugten Ausführungsform nur wenig (0,1 bis 0,5 Gew.%) und besonders bevorzugt keinen Fettalkohol.

Das Mittel erfüllt die an ein Haarkonditioniermittel hinsichtlich Konditionierwirkung zu stellenden Anforderungen in bester Weise. Das Haar ist nach der Behandlung sowohl im feuchten als auch im trockenen Zustand merkbar glatter und die Nass-kämmbarkeit ist merkbar verbessert. Überraschenderweise wurde gefunden, dass das Verdickungsmittel erlaubt, kationische Stoffe einzuarbeiten, ohne dabei negative Begleiteigenschaften des Verdickungsmittels zu fühlen. Die fachlichen Eigenschaften des erfindungsgemäßen Mittels übertreffen sogar noch die Eigenschaften einer konventionellen Haarkur auf Basis einer wäßrigen Emulsion von Fettalkoholen und quaternären Tensiden. Salon Tests im Halbseitenvergleich bestätigen dem erfindungsgemäßen Mittel eine bessere Kämmbarkeit und ein natürlicheres Anfühlen der Haare. Der meistens zu beobachtende negative stumpfe Griff der Haare von Fettalkohol-Kationtensid-Mischungen ist mit dem erfindungsgemäßen Mittel praktisch eliminiert. Die Haare fühlen sich leichter und unbelasteter an.

Die Mittel können zusätzlich amphotere Tenside in einer Menge von 0,1 bis 20 Gew.% enthalten. Amphotere Tenside sind Derivate aliphatischer quaternärer Ammonium-, Phosphonium- und Sulfoniumverbindungen der Formel 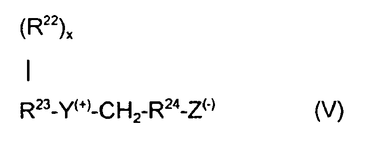 wobei R²² eine geradkettige oder verzweigtkettige Alkyl-, Alkenyl- oder Hydroxyalkylgruppe mit 8 bis 18 Kohlenstoffatomen und 0 bis etwa 10 Ethylenoxideinheiten und 0 bis 1 Glycerineinheiten darstellt; Y eine N-, P- oder S-haltige Gruppe ist; R²³ eine Alkyl- oder Monohydroxyalkylgruppe mit 1 bis 3 Kohlenstoffatomen ist; x gleich 1 ist, falls Y ein Schwefelatom ist und x gleich 2 ist, wenn Y ein Stickstoffatom oder ein Phosphoratom ist; R²⁴ eine Alkylen- oder Hydroxyalkylengruppe mit 1 bis 4 Kohlenstoffatomen ist und Z⁽⁻⁾ eine Carboxylat-, Sulfat-, Phosphonat- oder Phosphatgruppe darstellt.

Andere amphotere Tenside wie Betaine können ggf. ebenfalls in einer Menge von 0,1 bis 10 Gew.% in dem erfindungsgemäßen Mittel enthalten sein. Beispiele für Betaine umfassen C8- bis C18-Alkylbetaine wie Cocodimethylcarboxymethylbetain, Lauryldimethylcarboxymethylbetain, Lauryldimethylalphacarboxyethybetain, Cetyldimethylcarboxymethylbetain, Oleyldimethylgammacarboxypropylbetain und Lauryl-bis(2-hydroxypropyl)alphacarboxyethylbetain; C8- bis C18-Sulfobetaine wie Cocodimethylsulfopropylbetain, Stearyldimethylsulfopropylbetain, Lauryldimethylsulfoethylbetain, Laurylbis-(2-hydroxyethyl)sulfopropylbetain; die Carboxylderivate des Imidazols, die C8- bis C18-Alkyldimethylammoniumacetate, die C8- bis C18-Alkyldimethylcarbonylmethylammoniumsalze sowie die C8- bis C18-Fettsäurealkylamidobetaine wie beispielsweise das Kokosfettsäureamidopropylbetain, welches beispielsweise in Form einer 30%igen wäßrigen Lösung unter der Handelsbezeichnung Tego® Betain L7 von der Firma Goldschmidt AG vertrieben wird und das N-Kokosfettsäureamidoethyl-N-[2-(carboxymethoxy)ethyl]-glycerin (CTFA-Name: Cocoamphocarboxyglycinate), welches z.B. in Form einer 50%igen wäßrigen Lösung unter der Handelsbezeichnung Miranol® C2M von der Firma Miranol Chemical Co. Inc vertrieben wird.

Das Mittel kann weitere konditionierende Stoffe, insbesondere nichtionische Silikone mit Alkylenoxidgruppen wie Polydimethylsiloxane mit polyoxyalkylierten Substituenten, insbesondere Silikone, die mit Polypropylenoxid, Polyethylenoxid oder deren Gemisch modifiziert sind enthalten. Die Alkylenoxidgruppen können dabei seitenständig oder endständig sein oder es kann sich um Polydimethylsiloxan/Polyalkylenoxid Blockcopolymere handeln. Die mit Alkylenoxiden modifizierten Siloxane werden auch als Dimethylsiloxanglykolcopolymere oder als Dimethicon Copolyole bezeichnet. Geeignete Silikone mit Hydroxylgruppen sind Dimethiconole. Hierbei handelt es sich um Polydimethylsiloxane mit Hydroxylendgruppen. Geeignete Silikone mit Thiosulfatgruppen sind bekannt unter der INCI-Bezeichnung Dimethicone/Sodium PG-Propyldimethicone Thiosulfate Copolymer.

Das erfindungsgemäße Mittel kann weiterhin zusätzlich Träger- und Hilfsstoffe, wie zum Beispiel Lösungsmittel wie Wasser, niedere aliphatische Alkohole, beispielsweise Ethanol, n-Propanol und Isopropanol, Glykolether oder Glykole wie Glycerin, Ethylenglykol und insbesondere 1,2-Propandiol enthalten; der Wassergehalt beträgt vorzugsweise von 40 bis 95, besonders bevorzugt von 60 bis 90 Gewichtsprozent. Der Alkoholgehalt beträgt vorzugsweise von 1 bis 30, besonders bevorzugt von 5 bis 20 Gewichtsprozent. Besonders bevorzugt sind rein wäßrige Formulierungen.

In einer weiteren Ausführungsform enthält das erfindungsgemäße Mittel zusätzlich mindestens ein filmbildendes, haarfestigendes, synthetisches oder natürliches Polymer. Dieses zusätzliche Polymer kann nichtionischen, anionischen oder amphoteren Charakter haben und wird bevorzugt in einer Menge von 0,5 bis 5 Gew. % eingesetzt. Unter filmbildenden, haarfestigenden Polymeren werden solche Polymere verstanden, welche in 0,1 bis 5 %-iger wässriger, alkoholischer oder wäßrig-alkoholischer Lösung angewandt in der Lage sind, auf dem Haar einen Polymerfilm abzuscheiden und auf diese Weise das Haar zu festigen.

Das erfindungsgemäße Mittel kann darüber hinaus die für Haarbehandlungsmittel üblichen Zusatzbestandteile enthalten, z. B. nicht festigende nichtionische Polymere, nicht festigende anionische Polymere und nicht festigende natürliche Polymere sowie deren Kombination in einer Menge von vorzugsweise 0,01 bis 10 Gew. %; Parfümöle in einer Menge von vorzugsweise 0,01 bis 5 Gew. %; Netzmittel oder Emulgatoren aus den Klassen der anionischen, kationischen, amphoteren oder nichtionogenen oberflächenaktiven Substanzen in einer Menge von vorzugsweise 0.01 bis 10 Gew. %; Feuchthaltemittel; Konservierungsmittel, bakterizide und fungizide Wirkstoffe wie z.B. 2,4,4-Trichlor-2-hydroxydiphenylether, Parabene oder Methylchlorisothiazolinon, in einer Menge von 0,01 bis 1,0 Gew.%; Puffersubstanzen, wie z. B. Natriumcitrat, Natriumphosphat und Ammoniumhydrogencarbonat in einer Menge von 0,1 bis 1,0 Gew.%; Thiole, Ketocarbonsäuren (Oxocarbonsäuren), insbesondere α-Ketocarbonsäuren bzw. deren physiologisch verträgliche Salze in einer Menge von 0,1 bis 1,0 Gew.%; Anfärbefarbstoffe, wie z. B. Fluorescein Natriumsalz, in einer Menge von etwa 0,1 bis 1,0 Gew.%; Pflegestoffe, wie z. B. Pflanzen- und Kräuterextrakte, Protein- und Seidenhydrolysate, Lanolinderivate, in einer Menge von 0,1 bis 5 Gew.%; Lösungsvermittler, Netzmittel oder Emulgatoren aus den Klassen der amphoteren oder nichtionogenen oberflächenaktiven Substanzen wie, Alkylbetaine, ethoxylierte Fettalkohle, ethoxylierte Nonylphenole, Fettsäurealkanolamide, ethoxylierte Nonylphenole, ethoxylierte Fettsäureester in Konzentrationen von insgesamt 0,2 bis 30 Gewichtsprozent; ferner zusätzliche Verdicker wie Stärke oder Cellulosederivate in einer Menge von insgesamt 0,5 bis 5 Gewichtsprozent; UV-Absorber, Konditionierer, Haarquellmittel, Vaseline, Paraffinöl und Fettsäuren sowie außerdem Pflegestoffe wie Lanolinderivate, Cholesterin, Pantothensäure und Betain; Antioxidantien, Radikalfänger, Antischuppenwirkstoffe, Glanzgeber, Vitamine und rückfettende Agenzien in einer Menge von 0,01 bis 10 Gew.%; Treibmittel wie z. B. Propan, Butan, Pentan, Dimethylether, Stickstoff, N₂O und Kohlendioxid, in einer Menge von 1 bis 50 Gew.%, vorzugsweise 10 bis 20 Gew.%.

Bei der Behandlung keratinischer Fasern kann das Mittel dort (insbesondere im Haar) verbleiben oder nach der Anwendung ausgespült werden. Im letzteren Fall beträgt die Einwirkungszeit des Mittels, je nach Temperatur (etwa 20 bis 60 Grad Celsius, vorzugsweise 30 bis 50 Grad Celsius), 1 bis 60 Minuten, insbesondere 5 bis 20 Minuten, wobei durch Wärmezufuhr die Wirkung beschleunigt werden kann; daher ist die Anwendung von Wärme bevorzugt. Nach Beendigung der Einwirkungszeit kann das Haar mit Wasser gespült und gegebenenfalls mit einem Shampoo gewaschen werden.

Bei den für die erfindungsgemäßen Mittels handelt es sich vorzugsweise um Pflegecremes, Pflegepülungen, Kuren und Kurschäume. Das Mittel kann auch als pflegendes Vorbehandlungsmittel vor chemischen und/oder physikalischen Behandlungen von keratinischen Fasern, insbesondere einer Haarfärbung, einer Haartönung, einer Haarbleichung oder vor einer Haardauerverformung, verwendet werden, um einer Haarschädigung durch diese oxidativen Behandlungen vorzubeugen.

Das erfindungsgemäße Mittel kann in einem pH-Bereich von 2,0 bis 9,5 vorliegen. Besonders bevorzugt sind schwach saure pH-Werte im Bereich zwischen 4,5 und kleiner 7, besonders bevorzugt von 5,5 bis 6,5. Liegt das erfindungsgemäße Mittel im sauren Bereich vor, so kann es organische oder anorganische Säuren enthalten wie beispielsweise Ameisensäure, Weinsäure, Äpfelsäure, Maleinsäure, Fumarsäure, Glyoxylsäure, Pyrrolidoncarbonsäure, Zitronensäure, Milchsäure, Schwefelsäure, Essigsäure, Salzsäure, Phosphorsäure u.a..

Das erfindungsgemäße Mittel liegt bevorzugt als Creme vor, welche bevorzugt ein thixotropes Fliessverhalten zeigt.

Es kann zur Erreichung einer besonders guten Verteilbarkeit auf dem Haar auch versprüht werden. Das erfindungsgemäße Haarbehandlungsmittel liegt dann in Kombination mit einer geeigneten mechanisch betriebenen Sprühvorrichtung vor. Unter mechanischen Sprühvorrichtungen sind solche Vorrichtungen zu verstehen, welche das Versprühen einer verdickten Flüssigkeit ohne Verwendung eines Treibmittels ermöglichen. Als geeignete mechanische Sprühvorrichtung kann z.B. eine Sprühpumpe oder ein mit einem Sprühventil versehener elastischer Behälter, in dem das erfindungsgemäße kosmetische Mittel unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden.

Das erfindungsgemäße Mittel ist bevorzugt ein Haarbehandlungsmittel und ganz besonders bevorzugt ein kationisches Haarpflegemittel.

Das erfindungsgemäße kationische Haarpflegemittel wird angewendet, indem eine für den gewünschten Konditioniereffekt ausreichende Menge in oder auf dem trockenen Haar oder nach der Haarwäsche in oder auf dem nassen oder feuchten Haar verteilt wird. Die anzuwendende Menge hängt von der Haarfülle ab und beträgt typischerweise 1 bis 25 g. Bei der bevorzugten Verwendung als Rinse-Produkt wird nach einer ausreichenden Einwirkzeit von beispielsweise 1 bis 15 Minuten das Haar ausgespült. Anschließend wird das Haar gegebenenfalls durchgekämmt oder zur Frisur geformt und getrocknet. Bei einer Verwendung als Leave-in-Produkt wird das Haar nach Aufbringen des Mittels nicht ausgespült.

Das erfindungsgemäße Mittel wird kann auch als kationisches Haarfärbemittel oder Haartönungsmittel mit pflegenden Eigenschaften konfektioniert sein.

Ist das kationische Haarfärbmittel ein oxidatives Haarfärbemittel, soenthält es mindestens eine Kupplersubstanz und mindestens eine Entwicklersubstanz sowie gegebenenfalls zusätzlich mit sich selbst kuppelnde Farbvorstufen und direkt auf das Haar aufziehende Farbstoffe.

Die Entwicklersubstanzen und Kupplersubstanzen werden in den Haarfärbemitteln entweder in Form der freien Base oder in Form ihrer physiologisch unbedenklichen Salze mit anorganischen oder organischen Säuren, wie zum Beispiel als Chlorid, Sulfat, Phosphat, Acetat, Propionat, Ladat oder Citrat, eingesetzt.

Die Kupplersubstanzen und Entwicklersubstanzen werden vorzugsweise in etwa equimolarer Menge eingesetzt. Es ist jedoch nicht nachteilig, wenn die Kupplersubstanzen gegenüber den Entwicklersubstanzen in einem Überschuß oder Unterschuß verwendet werden, wobei sowohl die Entwicklerkomponente ein Gemisch von bekannten Entwicklersubstanzen als auch die Kupplerkomponente ein Gemisch von bekannten Kupplersubstanzen darstellen kann.

Als geeignete Entwicklersubstanzen sind beispielsweise zu nennen:
1,4-Diamino-benzol (p-Phenylendiamin), 1,4-Diamino-2-methyl-benzol (p-Toluylendiamin), 2,5-Diaminoanisol, 2,5-Diaminobenzylalkohol, 1,4-Diamino-2,6-dimethyl-benzol, 1,4-Diamino-2,5-dimethyl-benzol, 1,4-Diamino-2,3-dimethyl-benzol,2-Chlor-1,4-diaminobenzol, 4-Phenylamino-anilin, 4-Dimethylamino-anilin,4-Diethylamino-anilin, 4-[Di(2-hydroxyethyl)amino]-anilin,4-[(2-Methoxyethyl)amino]-anilin, 4-[(3-Hydroxypropyl)amino]-anilin, 1,4-Diamino-2-(2-hydroxyethyl)-benzol, 1,4-Diamino-2-(1-methylethyl)-benzol, 1,3-Bis[(4-aminophenyl)-(2-hydroxyethyl)amino]-2-propanol, 1,8-Bis(2,5-diaminophenoxy)-3,6-dioxaoctan, 4-Amino-phenol, 4-Amino-3-methyl-phenol, 4-Methylamino-phenol, 4-Amino-2-(aminomethyl)-phenol, 4-Amino-2-[(2-hydroxyethyl)-amino]methyl-phenol, 4-Amino-2-(methoxymethyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1H-pyrazol, 4,5-Diamino-1-(1-methylethyl)-1Hpyrazol, 4,5-Diamino-1-[(4-methylphenyl)methyl]-1H-pyrazol, 1-[(4-Chlorphenyl)methyl]-4,5-diamino-1H-pyrazol, 4,5-Diamino-1-methyl-1H-pyrazol, 2-Amino-phenol, 2-Amino-6-methyl-phenol und 2-Amino-5-methyl-phenol, allein oder im Gemisch miteinander.

Als geeignete Kupplersubstanzen sind beispielsweise zu nennen:
N-(3-Dimethylamino-phenyl)-harnstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)ö-amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methylphenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-2-methyl-phenol, 3-[(2-Hydroxyethyl)amino]-phenol, 3-[(2-Methoxyethyl)-amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxy-propyl)amino]-2-methyl-phenol, 3-[(2-Hydroxyethyl)amino]-2-methyl-phenol, 2-Amino-3-hydroxypyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxyphenol, 4-(β-Hydroxyethylamino)-1,2-methylendioxybenzol, 3,4-Methylendioxy-anilin, 5-[(2-Hydroxyethyl)amino]-1,3-benzodioxol, 5-Hydroxy-1,3-benzodioxol, 5-Amino-1,3-benzodioxol, 4-Methoxy-1-naphthol, 2-Methyl-1,3-dihydroxy-benzol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol, 2,3-Indolindion, 2,4-Dihydroxyphenolether wie 2,4-Dihydroxyanisol und 2,4-Dihydroxyphenoxyethanol.

Die Gesamtmenge der im kationischen Oxidationshaarfärbemittel enthaltenen Entwicklersubstanz-Kupplersubstanz-Kombination beträgt 0,01 bis 10,0 Gewichtsprozent, insbesondere 0,2 bis 4,0 Gewichtsprozent.

Zur Erzielung bestimmter Farbnuancen können ferner auch übliche direktziehende Farbstoffe, beispielsweise Triphenylmethanfarbstoffe wie Diamond Fuchsine (C.I. 42 510) und Leather Ruby HF (C.I. 42 520), aromatische Nitrofarbstoffe wie 2-Amino-4,6-dinitro-phenol, 2-Nitro-4-(n-hydroxyethylamino)-anilin, 2-N-β-Dihydroxypropylamino5-(N-methyl,N-hydroxyethyl)amino-nitrobenzol und 2-Amino-4-nitrophenol, Azofarbstofte wie Acid Brown 4 (C.I. 14 805) und Acid Blue 135 (C.I. 13 385), Anthrachinonfarbstoffe wie Disperse Violet 4 (C.I. 61 105), Disperse Blue 1 (C.I. 64 500), Disperse Red 15 (C.I. 60 710), Disperse Violet 1 (C.I. 61 100), 1,4,5,8-Tetraaminoanthrachinon und 1,4-Diaminoanthrachinon, in dem Haarfärbemittel enthalten sein.

Das Oxidationshaarfärbemittel kann weiterhin auch mit sich selbstkuppelnde Farbvorstufen, wie zum Beispiel 2-Amino-5-methylphenol, 2-Amino-6-methylphenol, 2-Amino-5-ethoxyphenol oder auch 2-Propyl-amino-5-aminopyridin, enthalten.

Die Gesamtmenge der direktziehenden Farbstoffe und der mit sich selbst kuppelnden Farbstoffvorstufen beträgt bevorzugt 0,01 bis 7,0 Gewichtsprozent, besonders bevorzugt 0,2 bis 4,0 Gewichtsprozent.

Die Anwendung des erfindungsgemäßen Oxidationshaarfärbemittels erfolgt indem man das cremeförmige Oxidationshaarfärbemittel unmittelbar vordem Gebrauch mit einerer Wasserstoffperoxidlösung oder -emulsion in einem Gewichtsverhältnis von 1 : 1 bis 1 : 4, vorzugsweise in einem Gewichtsverhältnis von 1 : 1 bis 1 : 3, vermischt, und sodann eine für die Haarfärbung ausreichende Menge des so erhaltenen gebrauchsfertigen Färbemittels, je nach Haarfülle im allgemeinen 60 bis 160 g, auf das Haar aufträgt. Nach einer Einwirkungszeit von etwa 5 bis 60 Minuten, vorzugsweise etwa 20 bis 30 Minuten, bei 15 bis 50 Grad Celsius wird das Haar mit Wasser ausgespült, gegebenenfalls mit einem Shampoo gewaschen und getrocknet. Falls erforderlich kann das Haar vor dem Trocknen zur Frisur gelegt werden.

Liegt das erfindungsgemäße Mittel als Haartönungsmittel mit pflegenden Eigenschaften vor, so enthält es direktziehende Farbstoffe, welche ausgewählt sind aus folgenden Gruppen:

### Nitrofarbstoffe (blau)

4-N-Ethyl-N-(2'-hydroxyethyl)amino-1-(2"-hydroxyethyl)amino-2-nitro-benzol, 1-Amino-3-methyl-4-(2'-hydroxyethyl)amino-6-nitro-benzol, 1-(2'-Hydroxyethyl)amino-2-nitro-4-bis-(2"-hydroxyethyl)amino-benzol, 4-Bis-(2'hydroxyethyl)amino-1-(2"-methoxyethyl)amino-nitrobenzol, 1-(2',3'-Dihydroxypropyl) amino-2-nitro-4-[ethyl-(2"-hydroxyethyl)aminol-benzol, 1-[(2',3'-Dihydroxypropyl)amino]-2-nitro-4-[ethyl-2"-(hydroxyethyl)amino]-benzol, 1-(3'-Hydroxypropylamino)-2-nitro-4-bis-(2"-hydroxyethylamino)-benzol.

### Nitrofarbstoffe (rot)

1-Amino-4-(2'-hydroxyethyl)-amino-nitrobenzol, 1-Hydroxy-2-amino-4,6-dinitrobenzol, 1-Amino-2-nitro-4-bis-(2'-hydroxyethyl)amino-benzol, 1-Amino-2-nitro-4-(2'hydroxyethyl)amino-5-chlorbenzol, 1-(2'Hydroxyethyl)amino-2-nitro-4-aminobenzol, 1 -Hydroxy-3-nitro-4-aminobenzol, 1 -(2'-Aminoethyl)amino-2-nitro-4-(2"hydroxyethyl)-oxybenzol, 3-Nitro-4-(2'-hydroxyethyl)amino-phenylglycerinether, 1-Amino-5-chlor-4-(2',3'-dihydroxypropyl)amino-2-nitro-benzol, 1,4-Bis-[(2',3'dihydroxypropyl)amino-5-chlor-2-nitro-benzol, 1-Hydroxy-2(2'hydroxyethyl)-amino-4,6-dinitro-benzol, 2-Amino-6-chlor-4-nitrophenol, 1-Hydroxy-3-nitro-4-(3'hydroxypropylamino)-benzol, 3-Nitro-4-ethylamino-benzoesäure, 4-Amino-2-nitrodiphenylamino-2-carbonsäure, 2-Chlor-6-ethylamino-4-nitrophenol, 2,5-Diamino-6-nitropyridin, 1,2,3,4-Tetrahydro-6-nitrochinoxalin.

### Nitrofarbstoffe (gelb)

4-(2'-Hydroxyethyl)amino-3-vitro-benzonitril, 4-(2'-Hydroxyethyl)amino-3-nitrobenzamid, 1 -Amino-2-(2'-hydroxyethyl)amino-5-nitro-benzol, 1-Methoxy-2-(2'hydroxyethyl)amino-5-nitro-benzol, 1-Hydroxy-3-nitro-4-(2'-hydroxyethyl)aminobenzol, 1-Hydroxy-2-amino-3-nitro-benzol, 1-Amino-2-methyl-6-nitro-benzol, 1-(2'-Hydroxyethyl)-oxy-3-methylamino-4-nitrobenzoi, 1-Methylamino-2-nitro-5-(2',3'-dihydroxypropyl)-oxybenzol, 1-(2'-Hydroxyethyl)amino-2-hydroxy-4-nitrobenzol, 1-Methoxy-3-(2'-aminoethyl)-amino-4-nitro-benzol, 1-(2'-Ureidoethyl)amino-4-nitro-benzol, 1-(2'-Hydroxyethyl)amino-2-nitro-benzol, 4-(2'-Hydroxyethyl)amino-3-nitrotrifluormethyl-benzol, 2,4-Bis-[N-(2'-hydroxyethyl)amino]-5-chlor-nitrobenzol, 4-(2',3'-Dihydroxypropyl)amino-3-nitro-trifluormethyl-benzol, 4-(2'-Hydroxymethyl)amino-3-nitro-methylbenzol, 4-(2'-Hydroxyethyl)amino-3-nitrochlorbenzol,

### Azofarbstoffe

1-(4'-Nitrophenylazo)-2-methyl-4-bis-(2"-hydroxyethyl)amino-benzol, 1-(3'-Nitro-4-amino)-phenylazo-2-hydroxy-7-trimethyl-ammoniumchlorid-naphthalin, 1-(2'-Hydroxy-4'-sulfo-6'-nitro)-naphthylazo-2-hydroxynaphthalin, 1-(4'-Aminophenylazo)-2-methyl-4-bis-[(2'-hydroxyethyl)-amino]-benzol, 5(4'-Dimethylaminophenylazo)-1,4-dimethyl-triazoniumchlorid, 1-(2'-Methoxyphenylazo)-2-hydroxy-7-trimethylammonium-naphthalinchorid, 1-(4'-Aminophenylazo)-2-hydroxy-7-trimethylammonium-naphthalin, 4-(3'-Trimethylammoniumphenylazo)-N-phenyl-3-methyl-pyrazolon (5), 4-Hydroxy-3-[(4'-sulfo-l'-naphthyl)azo]-1-naphthalinsulfonsäure, 1-(4'-Sulfophenylazo)-2-hydroxy-naphthalin, 1-(4'-Sulfophenylazo)-2-hydroxy-6-sulfo-naphthalin, 4-Amino-[4'-bis-(2"-hydroxyethyl)amino-azobenzol, 4-Amino-[4'-bis-(2"-hydroxyethyl)amino]-2'-methyl-azobenzol, 3-(2',6'-Diaminopyridyl-3'-azo)-pyridin, 7-Phenylazo-l-amino-3,6-disulfo-8-hydroxy-naphthalin, 5-Acetylamino-4-hydroxy-3-[(2'-methylphenyl)azo]-2,7-naphthalindisulfonsäure, 2-(2',4'-Dimethylphenylazo)-6-(4"-sulfophenylazo)-1,3-dihydroxybenzol.

### Chinonfarbstoffe

1,4-Bis-(2',3'-dihydroxypropyl)amino-anthrachinon, 1-Methylamino-4-(2'-hydroxyethyl)amino-anthrachinon, 2-(2'-Aminoethyl)amino-anthrachinon, 2-Brom-4,8-diamino-6-(3'-trimethylammonium)-phenylamino-1,5-naphthochinon, 1-(2'-Sulfo-4'methyl-phenyl)-amino-4-hydroxy-anthrachinon, 1,4-Diamino-anthrachinon, 1-Amino-2-sulfo-4-cyclo-hexylamino-anthrachinon, 1-Methylamino-4-aminopropylamino-anthrachinon, 1-Aminopropylamino-anthrachinon.

### Triphenylmethanfarbstoffe

4',4",4"'-Triamino-3-methyl-triphenylcarboniumchlorid, Bis-(4,4-Diethylaminophenyl)-4'-ethylanlino-naphthyl-carboniumchlorid, Bis-(4,4-Dimethylaminophen)-4'phenyl-amino-naphthyl-carboniumchlorid und 4,4-Bis-(N-Ethyl-3-sulfobenzyl)-amino-2"-sulfofuchsonium.

Auch natürliche Farbstoffe wie Henna, Indigo oder Yuglon sind geeignet.

Die direktziehenden Farbstoffe werden in dem Haarfärbe- bzw. Haartönungsmittel vorzugsweise in einer Gesamtmenge von 0,1 bis 10 Gewichtsprozent, besonders bevorzugt 0,3 bis 5 Gewichtsprozent, verwendet.

Gegenstand der Erfindung ist ferner die Verwendung von Inulin in einer Menge von 12 bis 60 Gew.% zur Verdickung von kosmetischen Mitteln, diemindestens einen kationischen Stoff, insbesondere ein kationisches Tensids und/oder kationisches Polymer, enthalten.

Die nachfolgenden Beispiele sollen den Gegenstand der Erfindung näher erläutern.

### Beispiel 1: Haarkur in Cremeform

| | |
|---|---|
| Cetyltrimethylammoniumchlorid | 1,0 Gew.% |
| diquaternäres Silikon, 50%ig in Propylenglykol (Abil® Quat 3272, CTFA: QUATERNIUM-80,) | 1,0 Gew.% |
| Inulin | 18,0 Gew.% |
| Silikonöl AK 500 | 1,0 Gew.% |
| Parfümöl | 0,1 Gew.% |
| Kreatin | 0,5 Gew.% |
| Wasser | ad 100,0 Gew.% |

### Beispiel 2: Haarkur in Cremeform

| | |
|---|---|
| Cetyltrimethylammoniumchlorid | 1,0 Gew.% |
| diquaternäres Silikon, 50%ig in Propylenglykol (Abil® Quat 3272, CTFA: QUATERNIUM-80,) | 1,0 Gew.% |
| Vinylpyrrolidon/Dimethylaminoethylmethacrylat-methosulfat Copolymer (Gafquat® 755N; CTFA: POLYQUATERNIUM-11) | 5,0 Gew.% |
| Inulin | 18,0 Gew.% |
| Silikonöl AK 500 | 1,0 Gew.% |
| Parfümöl | 0,1 Gew.% |
| Wasser | ad 100,0 Gew.% |

### Beispiel 3: Haarkur

| | |
|---|---|
| Cetyltrimethylammoniumchlorid | 2,0 Gew.% |
| Inulin | 20,0 Gew.% |
| Citronensäure | 0,1 Gew.% |
| Sorbinsäure | 0,1 Gew.% |
| Parfümöl | 0,1 Gew.% |
| Betain | 1,0 Gew.% |
| Wasser | ad 100,0 Gew.% |

### Beispiel 4: Haarkur

| | |
|---|---|
| Lauryltrimethylammoniumchlorid (Arquad® 12-50) | 0,5 Gew.% |
| diquaternäres Silikon, 50%ig in Propylenglykol (Abil® Quat 3272, CTFA: QUATERNIUM-80,) | 1,0 Gew.% |
| Inulin | 20,0 Gew.% |
| Cocomidopropylbetain (Tegobetain®) | 0,2 Gew.% |
| Glyoxylsäure | 0,2 Gew.% |
| Parfümöl | 0,1 Gew.% |
| Wasser | ad 100,0 Gew.% |

### Beispiel 5: kationische oxidative Haarfärbecreme

| | |
|---|---|
| Cetyltrimethylammoniumchlorid | 2,00 Gew.% |
| Inulin | 20,00 Gew.% |
| 3-Aminophenol | 0,10 Gew.% |
| Amino-4-[(2-hydroxyethyl)amino]-anisol | 0,04 Gew.% |
| Resorcin | 0,60 Gew.% |
| p-Toluylendiaminsulfat | 0,40 Gew.% |
| 2-Amino-5-methyl-phenol | 0,03 Gew.% |
| 4-Amino-3-methyl-phenol | 0,50 Gew.% |
| Citronensäure | 0,10 Gew.% |
| Sorbinsäure | 0,10 Gew.% |
| Parfümöl | 0,10 Gew.% |
| Betain | 1,00 Gew.% |
| Wasser | ad 100,00 Gew.% |

Man vermischt vor dem Gebrauch 20 g der Haarfärbecreme mit 40 g einer 8%igen wässrigen Wasserstoffperoxidlösung. Man trägt 60 g des gebrauchsfertigen Mittels auf mittelbraune, menschliche Haare auf und läßt das Gemisch 15 Minuten bei Raumtemperatur einwirken. Danach wird das Haarfärbegemisch mit Wasser ausgespült und das Haar getrocknet. Das so behandelte Haar ist vom Haaransatz bis zu den Haarspitzen in einem dunklen Goldblond gefärbt. Das Haar besitzt sowohl in nassem als auch im trockenen Zustand einen weichen und glatten Griff.

### Beispiel 6: Haartönungsmittel in Cremeform

| | |
|---|---|
| 1-Amino-4-(2'-hydroxyethyl)-amino-2-nitrobenzol (HC Red No. 7) | 0,19 Gew.% % |
| 1-Hydroxy-2-amino-4,6-dinitro-benzol, | 0,14 Gew.% |
| 4-Amino-2-nitro-diphenylamin (HC Red No. 1) | 0,12 Gew.% |
| Cetyltrimethylammoniumchlorid | 1,00 Gew.% |
| diquaternäres Silikon, 50%ig in Propylenglykol (Abil® Quat 3272, CTFA: QUATERNIUM-80,) | 1,00 Gew.% |
| Inulin | 18,00 Gew.% |
| Silikonöl AK 500 | 1,00 Gew.% |
| Parfümöl | 0,10 Gew.% |
| Kreatin | 0,50 Gew.% |
| Wasser | ad 100,00 Gew.% |

Man trägt 70g des obigen Haartönungsmittels auf mittellange hellbraune Haare auf und lässt es 20 Minuten lang einwirken. Nachdem das Haar gründlich mit Wasser gespült wurde, wird es getrocknet; es ist in einem kräftigen Rotton gefärbt.

## Patentansprüche

1. Kosmetisches Mittel mit einem Gehalt an
(A) 0,1 bis 10 Gew.% mindestens eines kationischen Tensids, der allgemeinen Formel
N⁽⁺⁾R₁R₂R₃R₄ • X⁽⁻⁾ (I),
wobei R₁ bis R₄ unabhängig voneinander aliphatische Gruppen, aromatische Gruppen, Alkoxygruppen, Polyoxyalkylengruppen, Hydroxyalkylgruppen, Arylgruppen oder Alkarylgruppen mit 1 bis 22 C-Atomen bedeuten und X⁽⁻⁾ ein Anion darstellt und/oder
mindestens eines kationischen Polymers ausgewählt aus kationaktiven Silikonverbindungen; Methylvinylimidazoliumchlorid/Vinylpyrrolidon Copolymeren; kationisch derivatisierten Proteinhydrolysaten, enthaltend eine oder zwei lange C8- bis C22-Alkylketten und entsprechend zwei oder eine kurze C1- bis C4-Alkylkette; Chitosan, Chitosansalzen und Chitosanderivaten und
(B) 12 bis 60 Gew.% Inulin
in einer geeigneten kosmetischen Basis.

2. Mittel nach Anspruch 1, **dadurch gekennzeichnet, dass** das Inulin in einer Menge von 15 bis 40 Gew.% enthalten ist.

3. Mittel nach einem der Ansprüche 1 bis 2, **dadurch gekennzeichnet, dass** das Inulin in einer Menge von 18 bis 25 Gew.% enthalten ist.

4. Mittel nach einem der Ansprüche 1 bis 3, **dadurch gekennzeichnet, dass** es keinen Fettalkohol enthält

5. Mittel nach einem der Ansprüche 1 bis 4, **dadurch gekennzeichnet, daß** es eine Creme ist.

6. Mittel nach Anspruch 5, **dadurch gekennzeichnet, dass** es die Creme ein thixotropes Fliessverhalten zeigt.

7. Mittel nach einem der Ansprüche 1 bis 6; **dadurch gekennzeichnet, daß** es ein Haarbehandlungsmittel ist.

8. Mittel nach Anspruch 7, **dadurch gekennzeichnet, daß** es eine kationisches Haarpflegemittel ist.

9. Verwendung von Inulin in einer Menge von 12 bis 60 Gew.% zur Verdickung von kosmetischen Mitteln, die mindestens ein kationisches Tensid und/oder mindestens ein kationisches Polymer nach Anspruch 1 enthalten.

## Claims

1. Cosmetic composition with a content of
(A) 0.1 to 10% by weight of at least one cationic surfactant of the general formula
N⁽⁺⁾R₁R₂R₃R₄ . X⁽⁻⁾ (I),
where R₁ to R₄, independently of one another, are aliphatic groups, aromatic groups, alkoxy groups, polyoxyalkylene groups, hydroxyalkyl groups, aryl groups or alkaryl groups having 1 to 22 carbon atoms, and X⁽⁻⁾ is an anion and/or
at least one cationic polymer selected from cation-active silicone compounds; methylvinylimidazolium chloride/vinylpyrrolidone copolymers; cationically derivatized protein hydrolyzates comprising one or two long C8 to C22-alkyl chains and correspondingly two or one short C1 to C4-alkyl chain; chitosan, chitosan salts and chitosan derivatives and
(B) 12 to 60% by weight of inulin
in a suitable cosmetic base.

2. Composition according to Claim 1, **characterized in that** the inulin is present in an amount from 15 to 40% by weight.

3. Composition according to one of Claims 1 to 2, **characterized in that** the inulin is present in an amount from 18 to 25% by weight.

4. Composition according to one of Claims 1 to 3, **characterized in that** it does not comprise a fatty alcohol.

5. Composition according to one of Claims 1 to 4, **characterized in that** it is a cream.

6. Composition according to Claim 5, **characterized in that** the cream exhibits thixotropic flow behaviour.

7. Composition according to one of Claims 1 to 6, **characterized in that** it is a hair treatment composition.

8. Composition according to Claim 7, **characterized in that** it is a cationic haircare composition.

9. Use of inulin in an amount from 12 to 60% by weight for thickening cosmetic compositions which comprise at least one cationic surfactant and/or at least one cationic polymer according to Claim 1.

## Revendications

1. Composition cosmétique ayant une teneur
(A) de 0,1 à 10 % en poids en au moins un tensioactif cationique de formule générale
N(⁺) R₁R₂R₃R₄. X⁽⁻⁾ (I)
dans laquelle R₁ à R₄ représentent, indépendamment les uns des autres, des groupes aliphatiques, des groupes aromatiques, des groupes alcoxy, des groupes polyoxyalkylène, des groupes hydroxyalkyle, des groupes aryle ou des groupes alkaryle, ayant de 1 à 22 atomes de carbone, et X⁽⁻⁾ représente un anion et/ou
au moins un polymère cationique choisi parmi des composés silicone cationiques, des copolymères chlorure de méthylvinylimidazolium/vinyl-pyrrolidone ; des hydrolysats de protéines transformés en dérivés cationiques, contenant une ou deux longue(s) chaîne (s) alkyle en C₈-C₂₂ et de façon correspondante deux ou une courte(s) chaîne (s) alkyle en C₁-C₄, le chitosane, des sels de chitosane et des dérivés de chitosane et
(B) de 12 à 60 % en poids en inuline
dans une base cosmétique appropriée.

2. Composition selon la revendication 1, **caractérisée en ce que** l'inuline est contenue en une quantité de 15 à 40 % en poids.

3. Composition selon la revendication 1 ou 2, **caractérisée en ce que** l'inuline est contenue en une quantité de 18 à 25 % en poids.

4. Composition selon l'une quelconque des revendications 1 à 3, **caractérisée en ce qu'**elle ne contient pas d'alcool gras.

5. Composition selon l'une quelconque des revendications 1 à 4, **caractérisée en ce qu'**elle est une crème.

6. Composition selon la revendication 5, **caractérisée en ce que** la crème présente un comportement d'écoulement thixotropique.

7. Composition selon l'une quelconque des revendications 1 à 6, **caractérisée en ce qu'**elle est une composition de traitement capillaire.

8. Composition selon la revendication 7, **caractérisée en ce qu'**elle est une composition cationique de soin capillaire.

9. Utilisation d'inuline en une quantité de 12 à 60 % en poids, pour l'épaississement de produits cosmétiques qui contiennent au moins un tensioactif cationique et/ou au moins un polymère cationique selon la revendication 1.
